# EUROPÄISCHE PATENTSCHRIFT

(11) **EP 3 548 871 B1**
(45) Veröffentlichungstag und Bekanntmachung des Hinweises auf die Patenterteilung: **30.03.2022**
(21) Anmeldenummer: 17809229.2
(22) Anmeldetag: 22.11.2017
(51) Int. Cl.: G01N 21/25

(54) **VORRICHTUNG ZUR AUFNAHME VOLLFLÄCHIGER BILDER EINER ZELLKULTURPLATTE MIT EINER ODER MEHREREN KAVITÄTEN**
DEVICE FOR CAPTURING FULL-SURFACE IMAGES OF A CELL CULTURE PLATE WITH ONE OR MORE CAVITIES
DISPOSITIF POUR ENREGISTRER DES IMAGES SUR TOUTE LA SURFACE D'UNE PLAQUE DE CULTURE CELLULAIRE

(30) Priorität: 30.11.2016 EP 16201303
(43) Veröffentlichungstag der Anmeldung: 09.10.2019
(73) Patentinhaber: Bayer Aktiengesellschaft, 51371 Leverkusen (DE)
(72) Erfinder: FOIS, Franco, 40789 Monheim (DE); HARNAU, Michael, 42799 Leichlingen (DE); OCHMANN, Klaus, 51377 Leverkusen (DE)
(74) Vertreter: BIP Patents
(86) Internationale Anmeldenummer: PCT/EP2017/080017
(87) Internationale Veröffentlichungsnummer: WO 2018/099778

(56) Entgegenhaltungen:
- WO-A1-2008/117031
- WO-A1-2016/116291
- US-A1- 2010 184 616

## Beschreibung

Die Erfindung betrifft eine Vorrichtung zur Ermittlung der Wirkung von Wirkstoffen an Nematoden und anderen Organismen in wässrigen Tests. Die Erfindung betrifft des Weiteren ein Verfahren zur Justierung einer Beleuchtungseinrichtung der Vorrichtung.

Viele Arten von Nematoden (Fadenwürmer) stellen Schädlinge in der Landwirtschaft dar, da sie durch ihr Eindringen in die Wurzelsysteme den Pflanzenstoffwechsel stark beeinträchtigen können. Gegen einen Nematodenbefall sind bereits verschiedene chemische Substanzen, die sogenannten Nematiziden, entwickelt worden. Jedoch besteht ein großer Bedarf, weitere Wirkstoffe zu identifizieren, durch die Nematoden wirkungsvoll bekämpft werden können.

Aus der WO 2016/116291 A1 ist eine Vorrichtung zur Ermittlung der Wirkung von Wirkstoffen an Nematoden bekannt, mit der eine Vielzahl von Wirkstoffen in kurzer Zeit getestet werden können. Dabei werden gleichzeitig die einzelnen mit Nematoden und unterschiedlichen Wirkstoffen befüllten Kavitäten einer Zellkulturplatte untersucht. Die durch eine Halterung der Vorrichtung gehaltene Zellkulturplatte weist dabei eine Unterseite, eine Oberseite sowie vier Seitenflächen auf, die sich zwischen der Unterseite und der Oberseite der Zellkulturplatte erstrecken. Die Vorrichtung umfasst eine Kamera, die dazu dient, Bilder von der Unterseite der Zellkulturplatte und damit allen Kavitäten gleichzeitig aufzunehmen. Eine Beleuchtungseinrichtung der Vorrichtung weist zwei sich gegenüberstehende Lichtquellen auf, die die Zellkulturplatte ausleuchten.

Es hat sich gezeigt, dass die Anwendung der Vorrichtung der WO 2016/116291 A1 bei der Untersuchung von sehr kleinen Fadenwürmen wie Dirofilarien ihre Grenzen findet. Die Auflösung der Vorrichtung lässt sich zwar grundsätzlich durch Auswahl einer entsprechend höher auflösenden Kamera steigern, doch hat sich herausgestellt, dass Ungleichmäßigkeiten in der Ausleuchtung der Zellkulturplatte bei größerer Auflösung softwaretechnisch nicht mehr in befriedigendem Maße korrigiert werden können. Eine zuverlässige und belastbare Ermittlung der Wirkung der Wirkstoffe ist somit bei sehr kleinen Organismen nicht mehr möglich.

Die WO 2008/117031 A1 beschreibt eine Vorrichtung zum Auslesen einer Mikroorganismen-Testplatte mittels einer Kamera. Die Testplatte wird durch vier Lichtpaneele beleuchtet, die Licht in die vier Seitenflächen der Testplatte einstrahlen, wobei die Intensität des von den Lichtpaneelen ausgestrahlten Lichts manuell oder mittels eines Computers einstellbar ist.

Der Erfindung liegt daher die Aufgabe zu Grunde, eine Vorrichtung zur Ermittlung der Wirkung von Wirkstoffen an Nematoden und anderen Organismen bereitzustellen, durch die auch bei sehr kleinen Organismen wie Dirofilarien eine zuverlässige Ermittlung der Wirkung der Wirkstoffe möglich ist.

Diese Aufgabe wird durch die Vorrichtung nach Anspruch 1 gelöst. Ausführungsbeispiele der Erfindung können den Unteransprüchen zu Anspruch 1 entnommen werden.

Die erfindungsgemäße Vorrichtung zeichnet sich dadurch aus, dass das Licht einer ersten Lichtquelle durch eine erste Seitenfläche, dass Licht einer zweiten Lichtquelle durch eine zweite Seitenfläche, das Licht einer dritten Lichtquelle durch eine dritte Seitenfläche und das Licht einer vierten Lichtquelle durch eine vierte Seitenfläche jeweils von außen in die Zellkulturplatte treten. Somit tritt von allen Seitenflächen Licht in das Innere der Zellkulturplatte ein. Durch diese Maßnahme ist grundsätzlich eine gleichmäßige Ausleuchtung der Zellkulturplatte im Sinne der Erfindung möglich. Bei entsprechend leistungsfähiger Kamera kann somit die Wirkung von Wirkstoffen auch auf sehr kleine Organismen in Zellkulturplatten untersucht werden. Somit können belastbare Ergebnisse bei einer Auflösung von 30 µm und kleiner erreicht werden. In einem Ausführungsbeispiel beträgt die Auflösung weniger als 20 µm, beispielsweise 15 µm.

Für die erste Lichtquelle kann eine Intensität des Lichts individuell eingestellt werden. Dies gilt sinngemäß auch für die zweite Lichtquelle, die dritte Lichtquelle und die vierte Lichtquelle. Beispielsweise kann somit die Intensität des Lichts der ersten Lichtquelle unabhängig von der Intensität des Lichts der zweiten Lichtquelle oder einer anderen Lichtquelle eingestellt werden. Letztlich können also die vier Lichtquellen mit jeweils unterschiedlichen Lichtintensitäten Licht in die Zellkulturplatte emittieren.

Die erste Lichtquelle kann seitlich neben der ersten Seitenfläche angeordnet sein, sodass in Einsatzlage der Zellkulturplatte (die Zellkulturplatte erstreckt sich dabei in einer horizontalen Ebene) ein Lichtstrahl der ersten Lichtquelle im Wesentlichen horizontal durch die erste Seitenfläche in die Zellkulturplatte gelangt. Etwaige Umlenkspiegel oder andere optische Umlenkungen können somit entfallen.

Die Ausführungen zu der ersten Lichtquelle sollen auch sinngemäß für die anderen Lichtquellen gelten. So kann beispielsweise auch die zweite Lichtquelle seitlich neben der zweiten Seitenfläche angeordnet sein. Auch die im Folgenden beschriebenen Merkmale, die sich auf die erste Lichtquelle beziehen, können bei einer Einzelnen der übrigen Lichtquellen, bei einigen der übrigen Lichtquellen oder auch bei allen übrigen Lichtquellen verwirklicht sein.

Die Erfindung geht von einer rechteckigen Zellkulturplatte aus, die eine viereckige Grundform mit jeweils zwei parallelen Seitenflächen aufweist. Beispielsweise kann das Verhältnis von langen Kanten (langen Seitenflächen) zu kurzen Kanten (kurze Seitenflächen) 3 zu 2 betragen. Die Kavitäten sind üblicherweise in mehreren, zueinander parallel angeordneten Reihen angeordnet. Beispielsweise kann eine Zellkulturplatte 8 Reihen mit jeweils 12 Kavitäten aufweisen. Typischerweise werden gemäß ANSI-Standard viereckigen Mikrotiterplatten mit 6, 12, 24, 96 bzw. 384 Kavitäten verwendet.

Der Erfindung liegt die Erkenntnis zu Grunde, dass bei der Untersuchung von sehr kleinen Organismen die erforderliche gute Ausleuchtung aller Kavitäten der Zellkulturplatte mit einer Beleuchtungseinrichtung möglich ist, wenn Licht von allen Seitenflächen in die Zellkulturplatte tritt. Bei einer sechseckigen Zellkulturplatte, deren entsprechende Grundform aber derzeit unüblich ist, müssten gemäß der Erfindung alle sechs Seitenflächen mit Licht bestrahlt werden. Bei einer dreieckigen Zellkulturplatte wäre die Verwendung von nur drei Lichtquellen ausreichend.

Die Seitenflächen erstrecken sich zwischen der Oberseite und der Unterseite der Zellkulturplatte, die lichtdurchlässige Wände aufweist.

Die erste Lichtquelle erstreckt sich über die gesamte erste Seitenfläche, wodurch eine gleichmäßige Bestrahlung der ersten Seitenfläche möglich ist.

Erfindungsgemäß lassen sich für die erste Lichtquelle eine vertikale Höhe bezogen auf die Zellkulturplatte, ein Neigungswinkel um eine eigene Längsachse und/oder ein horizontaler Abstand zu der ersten Seitenfläche durch Mittel zur Justierung der Beleuchtungseinrichtung (30) individuell einstellen.

Durch die flexible und individuell einstellbare Einstellung einer jeden Lichtquelle kann die Ausleuchtung der Zellkulturplatte optimiert werden. Jede Lichtquelle ist dabei räumlich gesehen dreidimensional verstellbar (Höhe, horizontaler Abstand, Neigungswinkel). Zusätzlich kann eine vierte Dimension eingestellt werden, nämlich die Lichtintensität. Es hat sich herausgestellt, dass auch bei einer symmetrischen Zellkulturplatte mit rechteckiger Grundform eine präzise individuelle Einstellung der Lichtquelle an jeder der Seitenflächen für die Qualität der Messungen von großer Bedeutung ist.

Erfindungsgemäß ist jede Lichtquelle mit einer Steuerung verbunden und auf einer Halterung montiert, die mit der oder einer separaten Steuerung verbunden ist.

Eine vertikale Höhe des aus der ersten Lichtquelle austretenden Lichtstrahls kann 2 bis 6 mm, vorzugsweise 3 bis 5 mm betragen. Bei seitlicher Anordnung der ersten Lichtquelle tritt ohne Neigungswinkel dieser Lichtstrahl horizontal durch die erste Seitenfläche, also zwischen Oberseite und Unterseite in die Zellkulturplatte ein.

Vorzugsweise ist ein Öffnungswinkel des Lichtstrahls sehr klein, damit beispielsweise eine Deckfolie, mit der die einzelnen Kavitäten der Zellkulturplatte abgedeckt sind, nicht ausgeleuchtet wird und somit keine Störeffekte hervorrufen kann. Jedoch können auch größere Werte für den Öffnungswinkel toleriert werden, wenn beispielsweise durch die Kamera nur ein kleiner vertikaler Abschnitt der Zellkulturplatte scharf abgebildet wird, so dass die Ebene der Zellkulturplatte, in der sich die Deckfolie befindet, nicht mehr scharf ist. In dieser Ausführungsform wird jede Einstrahlung so eingehalten, dass die Zellkulturplatte nur vom Boden bis zur durch das Objektiv definierten Tiefenschärfe beleuchtet wird. Der Deckel bzw. Schutzfolie wird vorzugsweise nicht beleuchtet, um Lichtreflexionen zu vermeiden.

Erfindungsgemäß ist die Steuerung dafür konfiguriert, ein Verfahren zur Justierung der Beleuchtungseinrichtung automatisch durchzuführen.

Die erste Lichtquelle kann eine Reihe von nebeneinander angeordneten Leuchtdioden (LED) aufweisen. Um über eine wirksame Länge der ersten Lichtquelle ein gleichmäßiges Licht zu erzeugen, kann die erste Lichtquelle eine Fresnel-Linsenanordnung aufweisen. Durch diese Fresnel-Linsenanordnung emittiert somit die erste Lichtquelle einen Lichtstrahl, dessen Intensität über die Längserstreckung der Lichtquelle gesehen konstant ist.

Die Halterung für die Zellkulturplatte (auch MTP-Halterung genannt) kann mehrere Aufnahmeecken aufweisen, wobei eine erste Aufnahmeecke zur Aufnahme eines Endes der ersten Seitenfläche und eine zweite Aufnahmeecke zur Aufnahme eines gegenüberliegenden Endes der ersten Seitenfläche dienen. Ein Abstand zwischen der ersten Aufnahmeecke und der zweiten Aufnahmeecke ist dabei vorzugsweise größer als eine Länge einer Reihe von Kavitäten, die sich entlang der ersten Seitenfläche erstreckt. Das Licht der ersten Lichtquelle, kann somit über die gesamte Länge der Reihe von Kavitäten ungehindert in die Zellkulturplatte eintreten. Vorzugsweise ist die Halterung so ausgebildet, dass zwischen der ersten Lichtquelle und der ersten Seitenfläche der Zellkulturplatte keine sonstigen Hindernisse angeordnet sind, um einen ungehinderten und nicht verschatteten Eintritt des Lichts der ersten Lichtquelle in die Zellkulturplatte zu ermöglichen.

Die Kamera kann ein telezentrisches Objektiv zum präzisen Messen (z.B. von Sill Optics) umfassen, um einen perspektivischen Fehler des Bildes an den Rändern der Zellkulturplatte auszuschließen oder zumindest weitestgehend auszuschließen.

Typischerweise wird eine Digitalkamera mit einem Bildsensor verwendet. Vorzugsweise wird der Boden der Zellkulturplatte zur Bildsensorfläche so justiert (auch Chip Tilt genannt) und die Zellkulturplatte zum Bildsensor so gedreht, dass die Zellkulturplatte möglichst genau parallel zum Bildsensor (bevorzugt Fläche und Seiten) justiert ist. Für die Justierung ist typischerweise die MTP-Halterung in allen Richtungen präzise beispielsweise mittels Klemmen mit definierter Stärke verstellbar.

Durch die Anwendung eines telezentrischen Objektives in Kombination mit einer verstellbaren auf MTP-Halterung montierten Zellplatte konnte eine Verzeichnung <+/- 2 %, d. h. eine flache Bildaufnahme ohne Software Korrektur erreicht werden. Durch die Blende des telezentrischen Objektivs kann zudem eine geringe Tiefenschärfe eingestellt werden, wodurch Störeffekte, beispielsweise verursacht durch die oben erwähnte Deckfolie für die Kavitäten, reduziert erreicht werden können.

Die erfindungsgemäße Vorrichtung ermöglicht die Bildanalyse von kompletten Plattenflächen und ist für Zellkulturplatten mit bis zu 384 Kavitäten mit kleinen Organismen wie z. B. Dirofilaria (Auflösungsgrenze circa 15 µm) geeignet. Offensichtlich können allgemein vollflächige Bilder von Zellkulturplatten enthaltend in Lösungen suspendierten Organismen bzw. Partikeln mit Hilfe der erfindungsgemäßen Vorrichtung aufgenommen und mittels Bildanalyse bearbeitet werden. Im Betrieb der Vorrichtung nimmt die Kamera von der gesamten Unterseite beispielsweise einer 96 Kavitäten umfassenden Zellkulturplatte in zeitlichen Abständen von beispielsweise 1 bis 5 Sekunden mehrere digitale Bilder auf. Durch eine Auswertung dieser Bilder anhand einer Pixelanalyse kann für jede einzelne

Kavität beispielsweise eine Kennzahl für die mittlere Geschwindigkeit ermittelt werden, mit der sich die in dieser Kavität befindlichen Dirofilarien bewegen. In einer Kavität mit einem wirksamen Wirkstoff ist die mittlere Geschwindigkeit dabei grundsätzlich kleiner als in einer Kavität ohne Wirkstoff. Auf Basis der mittleren Geschwindigkeit lässt sich somit die Wirksamkeit eines Wirkstoffes abschätzen. In diesem Zusammenhang wird auf die WO 2016/116291 A1 verwiesen, in der detailliert ein entsprechendes Verfahren zur Ermittlung der Wirkung von Wirkstoffen beschrieben wird, mit welchen die erfindungsgemäße Vorrichtung betrieben werden kann.

Bei einer ganz gleichmäßig ausgeleuchteten Zellkulturplatte und bei jeweils gleich gefüllten Kavitäten (beispielsweise jeweils ohne Wirkstoff) müsste sich theoretisch für jede Kavität eine gleiche mittlere Geschwindigkeit für die Bewegung der Dirofilarien ergeben, wenn die zwangsläufig vorhandene biologische Streuung außer Acht gelassen wird. Bei zu schlechter Ausleuchtung der Kavitäten weichen hingegen die Ergebnisse trotz gleicher Befüllung sehr deutlich voneinander ab. Bei einer derart ausgeleuchteten Zellkulturplatte mit unterschiedlichen Wirkstoffen befüllten Kavitäten wäre daher eine Aussage über die einzelnen Wirkstoffe nicht belastbar. Durch die Beleuchtungseinrichtung der erfindungsgemäßen Vorrichtung ist es jedoch möglich, den Einfluss der in der Praxis nicht ganz auszuschließenden Unterschiede in der Ausleuchtung so gering zu halten, dass eine belastbare Aussage über die Wirkung von Wirkstoffen auch bei sehr kleinen Dirofilarien (also bei entsprechend hoher Auflösung) möglich ist.

Darüber hinaus bestand die Aufgabe ein Verfahren zur Justierung der Beleuchtungseinrichtung der oben in ihren verschiedenen Ausführungen beschriebenen Vorrichtung bereit zu stellen. Diese Aufgabe wird durch die Merkmalskombination gemäß Anspruch 9 gelöst. Ein bevorzugtes Ausführungsbeispiel des erfindungsgemäßen Verfahrens kann dem Unteranspruch zu Anspruch 9 entnommen werden.

Das erfindungsgemäße Verfahren sieht vor, dass für jede der Lichtquellen die relative Höhe bezogen auf die Zellkulturplatte, der Neigungswinkel, der horizontale Abstand zur angrenzenden Seitenfläche sowie die Lichtintensität individuell eingestellt werden, um eine möglichst gleichmäßige Ausleuchtung der einzelnen Kavitäten der Zellkulturplatte zu erhalten.

In einem systematischen Prozess können iterativ einzelne Parameter (Höhe, Abstand, Neigungswinkel, Lichtintensität) für jede Lichtquelle eingestellt werden und mit diesen eingestellten Parametern jeweils eine oben beschriebene Ermittlung der mittleren Geschwindigkeit für jede gleich befüllte Kavität durchgeführt werden. Dieser Vorgang kann beliebig oft wiederholt werden. Je weniger die ermittelten Ergebnisse für die mittlere Geschwindigkeit dabei voneinander abweichen, desto geringer ist der Einfluss, den die nicht gleichmäßige Ausleuchtung der Zellkulturplatte auf die Versuchsergebnisse hat. Weichen die Ergebnisse schließlich nur noch in einer Größenordnung voneinander ab, die auf die biologische Streuung zurück zu führen ist, kann das Verfahren der Justierung beendet werden.

Erfindungsgemäß sieht das Verfahren zur Justierung der Beleuchtungseinrichtung vor, dass mit der Kamera ein Bild eines einfarbigen Blatts erstellt wird, dass sich in einer Ebene oberhalb einer Zellkulturplatte befindet (beispielsweise kann auf eine in der Halterung befindlichen nicht-gefüllten Zellkulturplatte unter Zwischenschaltung einer weiteren leeren Zellkulturplatte das Blatt angeordnet werden) und dass anhand einer Auswertung des Bildes die Justierung erfolgt bzw. verfeinert wird. Beispielsweise kann von dem Bild ein Histogramm der Lichtintensität erzeugt werden und auf Basis dieses Histogramms die Justierung erfolgen. Eine gleichmäßige Ausleuchtung des Blatts ist dann gegeben, wenn das dazugehörige Histogramm für alle Unterbereiche (dies können auch die einzelnen Pixel des digitalen Bildes sein) jeweils eine gleiche Lichtintensität ausweist, was bedeuten würde, dass das Histogramm aus lediglich einem Balken mit dieser einen Lichtintensität besteht.

In einem Ausführungsbeispiel wird die Beleuchtungseinrichtung justiert einerseits mittels der Ergebnisse einer Zellkulturplatte mit gleich befüllten Kavitäten und zusätzlich mittels des oben beschriebenen Blatts. Die (Erst-)Justierung anhand der gleich befüllten Zellkulturplatte und der (Nach-)Justierung durch das Blatt kann mehrmals hintereinander und/oder in umgekehrter Reihenfolge erfolgen.

Zudem kann am Ende einer Justierung die Lichtintensität der einzelnen Lichtquellen um jeweils einen gleichen Betrag oder proportional reduziert werden, so dass sich für die mittlere Lichtintensität des Bilds des einfarbigen Blatts Papier ein vorgegebener Wert (beispielsweise ein Mittelwert von 128, wenn die Lichtintensität Werte von 0 bis 256 annehmen kann). Im Betrieb der erfindungsgemäßen Vorrichtung wird dann jeder Kavität der Zellkulturplatte ein entsprechender Bereich auf dem Blatt Papier zugeordnet. Die durch die Kamera ermittelten Werte der Lichtintensität für die Pixel der Kavität können dann anhand der Lichtintensität des entsprechenden Bereichs auf dem Blatt Papier normiert werden. Ist beispielsweise für eine Kavität ein eher hellerer Bereich des Blatt Papiers ermittelt worden, werden die für diese Kavität ermittelten Werte für die Lichtintensität durch die Normierung reduziert.

Anhand eines in den Figuren dargestellten Ausführungsbeispiels soll die Erfindung näher erläutert werden. Es zeigen:
- Figur 1: schematisch den Aufbau einer erfindungsgemäßen Vorrichtung;
- Figur 2: schematisch eine **Zellkulturplatte** und eine Beleuchtungsvorrichtung mit vier Lichtquellen; und
- Figur 3: einen Schnitt entlang der Linie III-III in Figur 2.

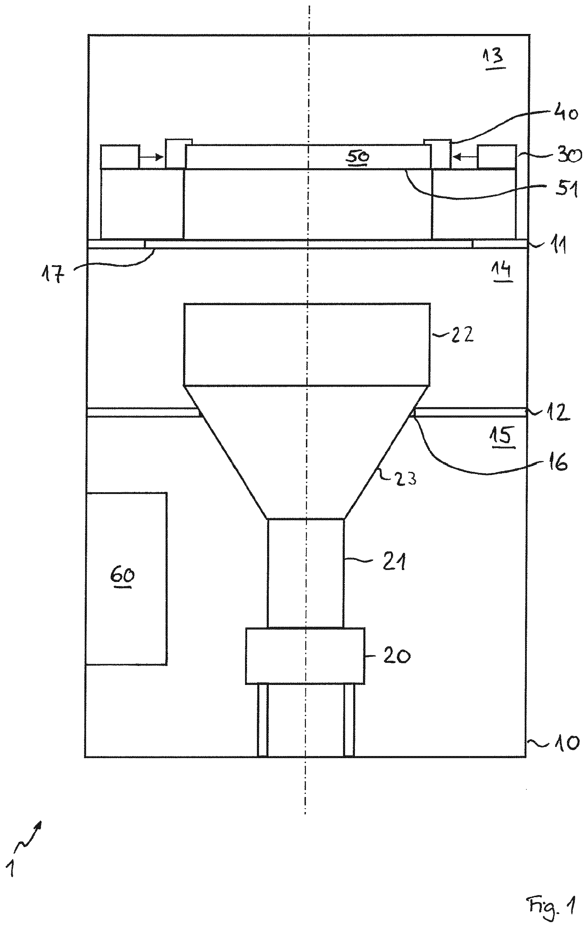

Figur 1 zeigt schematisch eine Vorrichtung zur Ermittlung der Wirkung von Wirkstoffen an Dirofilarien und anderen Organismen in wässrigen Tests. Die Vorrichtung, welche in ihrer Gesamtheit mit 1 bezeichnet wird, umfasst ein Gehäuse 10 mit einer oberen Trennplatte 11 und einer mittleren Trennplatte 12. Die Trennplatten 11, 12 unterteilen einen von dem Gehäuse 10 umgebenen Innenraum in einen oberen Bereich 13, einen mittleren Bereich 14 und einen unteren Bereich 15. In dem unteren Bereich 15 ist eine Kamera 20 montiert. Die Kamera 20 weist ein telezentrisches Objektiv 21 auf, dessen von der Kamera 20 abgewandtes Ende 22 in den mittleren Bereich 14 des Gehäuses 10 ragt. Die mittlere Trennplatte 12 weist eine kreisrunde Öffnung 16 auf, durch die das Objektiv 21 hindurchgreift. Ein kegelstumpfförmiger Abschnitt 23 des Objektivs 21 stützt sich dabei an dem kreisrunden Rand der Öffnung 16 ab.

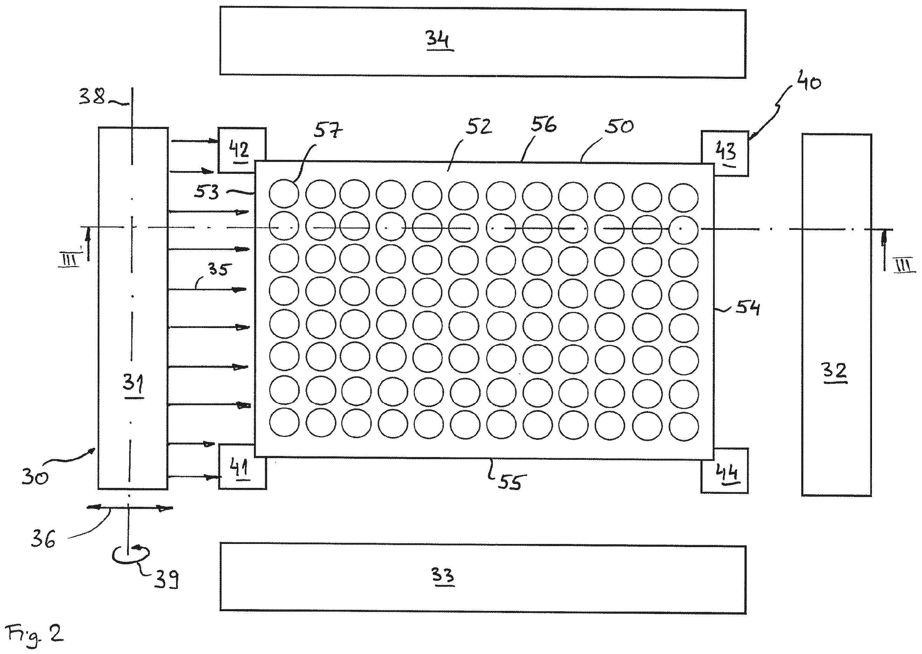

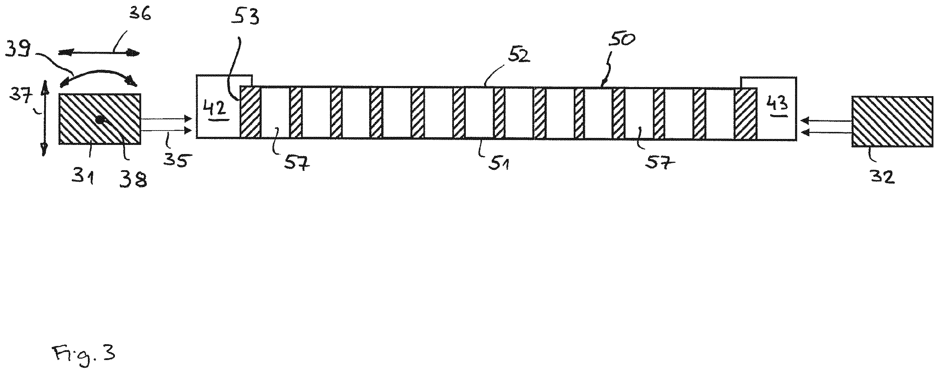

In dem oberen Bereich 13 des Gehäuses 10 ist eine Beleuchtungseinrichtung 30 sowie eine Halterung 40 für eine Zellkulturplatte 50 untergebracht. Die Beleuchtungseinrichtung 30, die Halterung 40 und die Zellkulturplatte 50 werden anhand der Figuren 2 und 3 im Folgenden noch näher beschrieben.

Neben der Kamera 20 nimmt der untere Bereich 15 des Gehäuses 10 auch eine Steuerung 60 für die Kamera 20 und die Beleuchtungseinrichtung 30 auf. Die Steuerung 60 kann dabei aus separaten Steuereinheiten für die Kamera 20 und die Beleuchtungseinrichtung 30 bestehen. Die Steuerung 60 lässt sich an einen Computer anschließen.

Mit der erfindungsgemäßen Vorrichtung 1 können von einer Unterseite 51 der Zellkulturplatte 50 erzeugt werden. In der Trennplatte 11 ist daher eine Glasscheibe 17 eingefasst.

Figur 2 zeigt von oben die Beleuchtungseinrichtung 30, die eine erste Lichtquelle 31, eine zweite Lichtquelle 32, eine dritte Lichtquelle 33 und eine vierte Lichtquelle 34 aufweist. Die erste Lichtquelle 31 umfasst mehrere Leuchtdioden (nicht dargestellt), die in einer Reihe nebeneinander angeordnet sind und einen über nahezu die gesamte Länge der ersten Lichtquelle 31 einen streifenförmigen Lichtstrahl 35 emittieren. Der Lichtstrahl ist durch die parallelen Pfeile 35 dargestellt. Auch die übrigen Lichtquellen 32, 33, 34 emittieren jeweils einen streifenförmigen Lichtstrahl über ihre nahezu vollständige Länge in Richtung der Zellkulturplatte 50. Der Übersicht halber sind aber entsprechende Pfeile zur Kennzeichnung der jeweiligen Lichtstrahlen in Figur 2 nicht dargestellt.

Die Zellkulturplatte 50 weist neben der oben bereits erwähnten Unterseite 51 eine Oberseite 52 sowie eine erste Seitenfläche 53, eine zweite Seitenfläche 54, eine dritte Seitenfläche 55 und eine vierte Seitenfläche 56 auf. Aufgrund der rechteckigen Grundform der Zellkulturplatte 50 verlaufen die sich gegenüberstehenden Seitenflächen 53, 54 senkrecht zu dem anderen Paar von parallel verlaufenden Seitenflächen 55, 56.

Die Zellkulturplatte 50 weist eine Vielzahl von Kavitäten 57 auf, die in 8 Reihenmit jeweils 12 Kavitäten angeordnet sind. Wie der Figur 3 entnommen werden kann, weist jede einzelne Kavität 57 im Längsschnitt eine U-förmige Form auf. Jede Kavität 57 lässt sich dabei von oben, also von der Oberseite 52 her mit einer wässrigen Lösung befüllen, in der sich die Dirofilarien und ein Wirkstoff befinden. Nach erfolgter Befüllung können die Kavitäten über eine Deckfolie abgedeckt werden.

Wie in der Zusammenschau der Figuren 2 und 3 deutlich wird, geben die einzelnen Lichtquellen 31, 32, 33, 34 jeweils Licht durch die Seitenflächen 53, 54, 55, 56 in die Zellkulturplatte 50 ab. So tritt Licht aus der ersten Lichtquelle von außen durch die erste Seitenfläche 53 in die Zellkulturplatte 50. Das gleiche gilt sinngemäß für die Lichtquellen 32, 33, 34. Um eine möglichst gute Ausleuchtung der einzelnen Kavitäten 57 der Zellkulturplatte 50 zu erreichen, lässt sich die Position der Lichtquelle 31 in Bezug auf die Zellkulturplatte 50 verändern.

Durch den Doppelpfeil 36 ist angedeutet, dass sich ein Abstand zwischen der ersten Lichtquelle 31 und der ersten Seitenfläche 53 der Zellkulturplatte 50 vergrößern oder verkleinern lässt. Der Doppelpfeil 37 (siehe Figur 3) zeigt an, dass sich die erste Lichtquelle 31 bezogen auf die Zellkulturplatte 50 nach oben oder nach unten bewegen lässt. Zudem kann die erste Lichtquelle 31 um eine Längsachse 38, die parallel zur ersten Seitenfläche 53 der Zellkulturplatte 50 verläuft, gekippt werden. Die entsprechende Verstellbarkeit um die Längsachse 38 oder um eine Achse, die parallel dazu verläuft, ist durch den gebogenen Pfeil 39 gekennzeichnet. Zudem lässt sich über die Steuerung 60 die Lichtintensität der ersten Lichtquelle 31 einstellen. Der horizontale Abstand zu der ersten Seitenfläche, die vertikale Lage, der Neigungswinkel und die Lichtintensität der ersten Lichtquelle können von den entsprechenden Einstellparametern der übrigen Lichtquellen 32, 33, 34 individuell eingestellt werden. Somit ist es beispielsweise möglich, die Beleuchtungseinrichtung derart zu justieren, dass die Lichtintensität der ersten Lichtquelle 31 nicht nur von den Lichtintensitäten der dazu quer angeordneten Lichtquellen 33, 34 abweicht, sondern auch von der Lichtintensität der gegenüberliegenden Lichtquelle 32.

Die Halterung 40 weist eine erste Aufnahmeecke 41, eine zweite Aufnahmeecke 42, eine dritte Aufnahmeecke 43 und eine vierte Aufnahmeecke 44 auf. Vorzugsweise sind dabei drei der vier Aufnahmeecken 41, 42, 43, 44 als Festanschläge ausgebildet, während die verbleibende vierte Aufnahmeecke als gefederter Anschlag ausgebildet ist. Ein Abstand zwischen beispielsweise der ersten Aufnahmeecke und der zweiten Aufnahmeecke ist so bemessen, dass der Lichtstrahl 35 der ersten Lichtquelle 31 ungehindert auf den Bereich der ersten Seitenfläche treten kann, in dem sich die Kavitäten 57 befinden. Mit anderen Worten ist ein Abstand zwischen den Aufnahmeecken 41, 42 größer als eine Länge der ersten Spalte der Kavitäten, die sich entlang der ersten Seitenfläche 53 erstreckt.

Aufgrund der unterschiedlichen Längen der längeren Seitenflächen 55, 66 und der kürzeren Seitenflächen 53, 54 sind auch die Leuchtquellen 33, 34 einerseits und die Lichtquellen 31, 32 andererseits unterschiedlich lang. Die Breite eines Lichtstrahls aus einer Lichtquelle ist dabei immer länger als die Länge des Bereichs der zugehörigen Seitenfläche, in dem sich die Kavitäten befinden.

Durch die Beleuchtungseinrichtung 30 und die besondere Anordnung der einzelnen Aufnahmeecken der Halterung 40 werden alle Bereiche der Seitenflächen der Zellkulturplatte 50, in denen sich die Kavitäten 57 befinden, mit Licht aus den Lichtquellen beaufschlagt. Durch die individuelle Einstellbarkeit der einzelnen Lichtquellen lässt sich die Beleuchtungseinrichtung 30 so justieren, dass eine annähernd optimale Ausleuchtung der Kavitäten 57 erreicht wird. Ein bevorzugtes Kontrollkriterium für die möglichst optimale Ausleuchtung ist oben bereits beschrieben. Von einer optimalen Ausleuchtung ist dann auszugehen, wenn bei einer Zellkulturplatte, deren einzelne Kavitäten mit jeweils dem gleichen Wirkstoff befüllt worden sind, die kavitätsspezifischen Ergebnisse hinsichtlich der Wirksamkeit des jeweils gleichen Wirkstoffes nicht voneinander abweichen bzw. eine Standardabweichung aufweisen, die in etwa der geschätzten Standardabweichung aufgrund der biologischer Organismen entspricht.

### Bezugszeichenliste

- 1: Vorrichtung

- 10: Gehäuse
- 11: obere Trennplatte
- 12: mittlere Trennplatte
- 13: oberer Bereich
- 14: mittlerer Bereich
- 15: unterer Bereich
- 16: Öffnung
- 17: Glasplatte

- 20: Kamera
- 21: Objektiv
- 22: Ende
- 23: kegelstumpfförmiger Bereich

- 30: Beleuchtungseinrichtung
- 31: erste Lichtquelle
- 32: zweite Lichtquelle
- 33: dritte Lichtquelle
- 34: vierte Lichtquelle
- 35: Pfeil (Lichtstrahl)
- 36: Doppelpfeil
- 37: Doppelpfeil
- 38: Pfeil

- 40: Halterung
- 41: erste Aufnahmeecke
- 42: zweite Aufnahmeecke
- 43: dritte Aufnahmeecke
- 44: vierte Aufnahmeecke

- 50: Zellkulturplatte
- 51: Unterseite
- 52: Oberseite
- 53: erste Seitenfläche
- 54: zweite Seitenfläche
- 55: dritte Seitenfläche
- 56: vierte Seitenfläche
- 57: Kavität

- 60: Steuerung

## Patentansprüche

1. Vorrichtung (1) zur Aufnahme von vollflächigen Bildern einer Zellkulturplatte mit einer oder mehreren Kavitäten, umfassend
- eine Halterung (40) zur Aufnahme der Zellkulturplatte (50) mit Kavitäten (57), wobei die Zellkulturplatte (50) eine Unterseite (51), eine Oberseite (62) sowie mehrere Seitenflächen aufweist, die sich zwischen der Unterseite (51) und der Oberseite (52) erstrecken,
- eine Kamera (20), die dazu dient, Bilder von der Unterseite (51) der Zellkulturplatte (50) innerhalb einer vordefinierten Tiefenschärfe aufzunehmen,
- eine Beleuchtungseinrichtung (30) zur Ausleuchtung der Zellkulturplatte (50), wobei die Beleuchtungseinrichtung (30) mehrere auf Halterungen montierte Lichtquellen zur Erzeugung von Lichtstrahlen umfasst, wobei in Einsatzlage der Zellkulturplatte (50)
- das Licht einer ersten Lichtquelle (31) durch eine erste Seitenfläche (53) über ihre gesamte Länge,
- das Licht einer zweiten Lichtquelle (32) durch eine zweite Seitenfläche (54) über ihre gesamte Länge,
- das Licht einer dritten Lichtquelle (33) durch eine dritte Seitenfläche (55) über ihre gesamte Länge, und
- das Licht einer vierten Lichtquelle (34) durch eine vierte Seitenfläche (56) über ihre gesamte Länge von außen in die Zellkulturplatte (50) tritt,
- wobei eine Intensität der erzeugten Lichtstrahlen, eine vertikale Höhe der Lichtquellen bezogen auf die Zellkulturplatte, ein horizontaler Abstand der Lichtquellen zu der jeweiligen Seitenfläche und ein Neigungswinkel der Lichtquellen um ihre Längsachse (38) für jede Lichtquelle (31) individuell einstellbar sind, und
- eine oder mehrere Steuerungen (60) für die Kamera (20) und die Beleuchtungseinrichtung (30), wobei jede Lichtquelle und jede Halterung mit der einen oder mehreren Steuerungen verbunden sind und wobei die eine oder mehreren Steuerungen dafür konfiguriert sind, ein Verfahren zur Justierung der Beleuchtungseinrichtung (30) automatisch durchzuführen, bei welchem mit der Kamera (20) ein Bild eines einfarbigen Blatts, das sich in einer Ebene oberhalb der ausgeleuchteten Zellkulturplatte (50) befindet, erstellt wird und anhand einer Auswertung des Bilds eine Justierung der vertikalen Höhe, des Neigungswinkels, des horizontalen Abstands und der Lichtintensität für jede Lichtquelle so erfolgt, dass eine gute Ausleuchtung der einzelnen Kavitäten (57) der Zellkulturplatte (50) sowie eine gleichmäßige Ausleuchtung der Zellkulturplatte erhalten wird.

2. Vorrichtung (1) nach Anspruch 1, wobei die erste Lichtquelle (31) eine Fresnel-Linsenanordnung aufweist, um über die wirksame Länge der ersten Lichtquelle (31) ein gleichmäßiges Licht zu erzeugen.

3. Vorrichtung (1) nach einem der Ansprüche 1 bis 2, wobei die erste Lichtquelle (31) seitlich neben der ersten Seitenfläche (53) angeordnet ist.

4. Vorrichtung (1) nach einem der Ansprüche 1 bis 3, wobei die Intensität der erzeugten Lichtstrahlen, die vertikale Höhe der Lichtquellen bezogen auf die Zellkulturplatte, der horizontale Abstand der Lichtquellen zu der jeweiligen Seitenfläche und der Neigungswinkel der Lichtquellen um ihre Längsachse (38) für jede Lichtquelle (31) so einstellbar sind, dass die Zellkulturplatte vom Boden bis zur vordefinierten Tiefenschärfe beleuchtet ist.

5. Vorrichtung (1) nach einem der Ansprüche 1 bis 4, wobei eine vertikale Höhe eines aus der ersten Lichtquelle (31) austretenden Lichtstrahls 2 bis 6 mm beträgt.

6. Vorrichtung (1) nach einem der Ansprüche 1 bis 5, wobei die Halterung (40) mehrere Aufnahmeecken aufweist, wobei eine erste Aufnahmeecke (41) zur Aufnahme eines Endes der ersten Seitenfläche (53) und eine zweiten Aufnahmeecke (42) zur Aufnahme eines gegenüberliegenden Endes der ersten Seitenfläche (53) dient, und wobei ein Abstand zwischen der ersten Aufnahmeecke (41) und der zweiten Aufnahmeecke (42) größer ist als eine Länge einer Reihe oder Spalte von Kavitäten (57), die sich entlang der ersten Seitenfläche (53) erstreckt.

7. Vorrichtung (1) nach einem der Ansprüche 1 bis 6, wobei die Kamera (20) ein telezentrisches Objektiv (21) umfasst.

8. Vorrichtung (1) nach einem der Ansprüche 1 bis 7, wobei die Kamera einen Bildsensor umfasst, und die Halterung (40) zur Aufnahme der Zellkulturplatte (50) und / oder der Bildsensor in allen Richtung zueinander verstellbar sind, so dass die Zellkulturplatte in allen Richtungen parallel zum Bildsensor justiert ist.

9. Automatisches Verfahren zur Justierung der Beleuchtungseinrichtung (30) der Vorrichtung (1) nach einem der Ansprüche 1 bis 8, wobei bezogen auf die Zellkulturplatte die vertikale Höhe einer jeden Lichtquelle, der horizontale Abstand einer jeden Lichtquelle zu der jeweiligen Seitenfläche und der Neigungswinkel einer jeden Lichtquelle um ihre Längsachse sowie eine Lichtintensität einer jeden Lichtquelle individuell eingestellt werden, um eine gute Ausleuchtung der einzelnen Kavitäten (57) der Zellkulturplatte (50) sowie eine gleichmäßige Ausleuchtung der Zellkulturplatte zu erhalten, indem mit der Kamera (20) ein Bild eines einfarbigen Blatts erstellt wird, das sich in einer Ebene oberhalb der ausgeleuchteten Zellkulturplatte (50) befindet, und anhand einer Auswertung des Bilds die Justierung erfolgt.

10. Verfahren nach Anspruch 9, wobei von dem Bild ein Histogramm der Lichtintensität erzeugt wird und auf Basis dieses Histogramms die Justierung durch Steuerung der Mittel zur Justierung der Beleuchtungseinrichtung (30) erfolgt, bis das Histogramm eine minimale Breite ausweist.

## Claims

1. Apparatus (1) for recording full-surface images of a cell culture plate with one or more wells, comprising
- a holder (40) for receiving the cell culture plate (50) with wells (57), wherein the cell culture plate (50) has a bottom side (51), a top side (62) and a plurality of side faces, which extend between the bottom side (51) and the top side (52),
- a camera (20) that serves to record images of the bottom side (51) of the cell culture plate (50) within a predefined depth of field,
- an illumination device (30) for illuminating the cell culture plate (50),
wherein the illumination device (30) comprises a plurality of light sources for producing light beams, said light sources being mounted on holders, wherein, in the use position of the cell culture plate (50),
- the light of a first light source (31) enters the cell culture plate (50) from the outside through the entire length of a first side face (53),
- the light of a second light source (32) enters the cell culture plate (50) from the outside through the entire length of a second side face (54),
- the light of a third light source (33) enters the cell culture plate (50) from the outside through the entire length of a third side face (55), and
- the light of a fourth light source (34) enters the cell culture plate (50) from the outside through the entire length of a fourth side face (56),
wherein an intensity of the produced light beams, a vertical height of the light sources in relation to the cell culture plate, a horizontal distance of the light sources from the respective side face and an inclination angle of the light sources about their respective longitudinal axis (38) for each light source (31) are individually adjustable, and
- one or more controllers (60) for the camera (20) and the illumination device (30), wherein each light source and each holder are connected to the one or more controllers and wherein the one or more controllers are configured to automatically carry out a method for adjusting the illumination device (30), within the scope of which the camera (20) is used to create an image of a monochrome sheet that is located in a plane above the illuminated cell culture plate (50) and an evaluation of the image is used to adjust the vertical height, the inclination angle, the horizontal distance and the luminous intensity for each light source such that a good illumination of the individual wells (57) of the cell culture plate (50) and a uniform illumination of the cell culture plate are obtained.

2. Apparatus (1) according to Claim 1, wherein the first light source (31) has a Fresnel lens arrangement in order to produce a uniform light over the effective length of the first light source (31).

3. Apparatus (1) according to either of Claims 1 and 2, wherein the first light source (31) is arranged laterally next to the first side face (53).

4. Apparatus (1) according to any one of Claims 1 to 3, wherein the intensity of the produced light beams, the vertical height of light sources in relation to the cell culture plate, the horizontal distance of the light sources from the respective side face and the inclination angle of the light sources about their respective longitudinal axis (38) are adjustable for each light source (31) in such a way that the cell culture plate is illuminated from the bottom to the predefined depth of field.

5. Apparatus (1) according to any one of Claims 1 to 4, wherein a vertical height of a light beam emerging from the first light source (31) is 2 to 6 mm.

6. Apparatus (1) according to any one of Claims 1 to 5, wherein the holder (40) has a plurality of receiving corners, wherein a first receiving corner (41) serves to receive one end of the first side face (53) and a second receiving corner (42) serves to receive an opposite end of the first side face (53), and wherein a distance between the first receiving corner (41) and the second receiving corner (42) is greater than a length of a row or column of wells (57), which extends along the first side face (53).

7. Apparatus (1) according to any one of Claims 1 to 6, wherein the camera (20) comprises a telecentric lens (21) .

8. Apparatus (1) according to any one of Claims 1 to 7, wherein the camera comprises an image sensor, and the holder (40) for receiving the cell culture plate (50) and/or the image sensor are adjustable in relation to one another in all directions such that the cell culture plate is adjusted parallel to the image sensor in all directions.

9. Automated method for adjusting the illumination device (30) of the apparatus (1) according to any one of Claims 1 to 8, wherein, in relation to the cell culture plate, the vertical height of each light source, the horizontal distance of each light source from the respective side face and the inclination angle of each light source about its longitudinal axis, and also a luminous intensity of each light source are set individually in order to obtain a good illumination of the individual wells (57) of the cell culture plate (50) and a uniform illumination of the cell culture plate by virtue of using the camera (20) to create an image of a monochrome sheet that is located in a plane above the illuminated cell culture plate (50) and the adjustment being implemented on the basis of an evaluation of the image.

10. Method according to Claim 9, wherein a histogram of the luminous intensity is produced from the image and the adjustment by way of controlling the means for adjusting the illumination device (30) is implemented on the basis of this histogram until the histogram has a minimum width.

## Revendications

1. Dispositif (1) d'acquisition d'images pleine surface d'une plaque de culture cellulaire pourvue d'une ou plusieurs cavités, ledit dispositif comprenant
- un support (40) destiné à recevoir la plaque de culture cellulaire (50) pourvue de cavités (57), la plaque de culture cellulaire (50) comportant une face inférieure (51), une face supérieure (62) et une pluralité de surfaces latérales qui s'étendent entre la face inférieure (51) et la face supérieure (52),
- une caméra (20) qui sert à acquérir des images depuis la face inférieure (51) de la plaque de culture cellulaire (50) dans une profondeur de champ prédéfinie,
- un moyen d'éclairage (30) destiné à éclairer la plaque de culture cellulaire (50), le moyen d'éclairage (30) comprenant une pluralité de sources de lumière montées sur des supports et destinées à générer des faisceaux lumineux,
lors de l'utilisation de la plaque de culture cellulaire (50)
- la lumière d'une première source de lumière (31) passant à travers une première surface latérale (53) sur toute sa longueur,
- la lumière d'une deuxième source de lumière (32) passant à travers une deuxième surface latérale (54) sur toute sa longueur,
- la lumière d'une troisième source de lumière (33) passant à travers une troisième surface latérale (55) sur toute sa longueur, et
- la lumière d'une quatrième source de lumière (34) passant à travers une quatrième surface latérale (56) sur toute sa longueur,
et ce depuis l'extérieur jusque dans la plaque de culture cellulaire (50),
- une intensité des faisceaux lumineux générés, une hauteur verticale des sources de lumière par rapport à la plaque de culture cellulaire, une distance horizontale des sources de lumière par rapport à la surface latérale respective et un angle d'inclinaison des sources de lumière sur leur axe longitudinal (38) pouvant être réglés individuellement pour chaque source de lumière (31),
et
- une ou plusieurs commandes (60) destinées à la caméra (20) et au moyen d'éclairage (30), chaque source de lumière et chaque support étant reliés à l'une ou plusieurs commandes et l'une ou plusieurs commandes étant configurées pour mettre en œuvre automatiquement un procédé de réglage du moyen d'éclairage (30), procédé dans lequel la caméra (20) permet de créer une image d'une feuille monochrome située dans un plan au-dessus de la plaque de culture cellulaire (50) éclairée et un réglage de la hauteur verticale, de l'angle d'inclinaison, de la distance horizontale et de l'intensité lumineuse étant effectué pour chaque source de lumière sur la base d'une évaluation de l'image de manière à obtenir un bon éclairage des cavités individuelles (57) de la plaque de culture cellulaire (50) et un éclairage uniforme de la plaque de culture cellulaire.

2. Dispositif (1) selon la revendication 1, la première source de lumière (31) comprenant un ensemble de lentilles de Fresnel pour générer une lumière uniforme sur la longueur efficace de la première source de lumière (31) .

3. Dispositif (1) selon l'une des revendications 1 et 2, la première source de lumière (31) étant disposée latéralement à côté de la première surface latérale (53).

4. Dispositif (1) selon l'une des revendications 1 à 3, l'intensité des faisceaux lumineux générés, la hauteur verticale des sources de lumière par rapport à la plaque de culture cellulaire, la distance horizontale des sources de lumière par rapport à la surface latérale respective et l'angle d'inclinaison des sources de lumière sur leur axe longitudinal (38) pouvant être réglés pour chaque source de lumière (31) de sorte que la plaque de culture cellulaire soit éclairée depuis le fond jusqu'à la profondeur de champ prédéfinie.

5. Dispositif (1) selon l'une des revendications 1 à 4, une hauteur verticale d'un faisceau lumineux sortant de la première source de lumière (31) étant de 2 à 6 mm.

6. Dispositif (1) selon l'une des revendications 1 à 5, le support (40) comportant une pluralité de coins de réception, un premier coin de réception (41) servant à recevoir une extrémité de la première surface latérale (53) et un deuxième coin de réception (42) servant à recevoir une extrémité opposée de la première surface latérale (53), et une distance entre le premier coin de réception (41) et le deuxième coin de réception (42) étant supérieure à une longueur d'une rangée ou d'une colonne de cavités (57) qui s'étend le long de la première surface latérale (53).

7. Dispositif (1) selon l'une des revendications 1 à 6, la caméra (20) comprenant un objectif télécentrique (21) .

8. Dispositif (1) selon l'une des revendications 1 à 7, la caméra comprenant un capteur d'image, et le support (40) destiné à recevoir la plaque de culture cellulaire (50) et/ou le capteur d'image étant réglables dans toutes les directions les uns par rapport aux autres de sorte que la plaque de culture cellulaire soit réglée parallèlement au capteur d'image dans toutes les directions.

9. Procédé automatique de réglage du moyen d'éclairage (30) du dispositif (1) selon l'une des revendications 1 à 8, par rapport à la plaque de culture cellulaire la hauteur verticale de chaque source de lumière, la distance horizontale de chaque source de lumière par rapport à la surface latérale respective et l'angle d'inclinaison de chaque source de lumière sur son axe longitudinal et une intensité lumineuse de chaque source de lumière étant réglés individuellement, afin d'obtenir un bon éclairage des cavités (57) individuelles de la plaque de culture cellulaire (50) et un éclairage uniforme de la plaque de culture cellulaire par le fait que la caméra (20) permet de créer une image d'une feuille monochrome qui est située dans un plan au-dessus de la plaque de culture cellulaire (50) éclairée et le réglage étant effectué sur la base d'une évaluation de l'image.

10. Procédé selon la revendication 9, un histogramme de l'intensité lumineuse étant généré à partir de l'image et le réglage étant effectué sur la base de cet histogramme par commande des moyens de réglage du moyen d'éclairage (30) jusqu'à ce que l'histogramme ait une largeur minimale.
